# EUROPEAN PATENT APPLICATION

(11) **EP 2 977 753 A1**
(43) Date of publication of application: **27.01.2016**
(21) Application number: 14767635.7
(22) Date of filing: 20.02.2014
(51) Int. Cl.: G01N 27/447, C12M 1/00, G01N 37/00

(54) **ELECTROPHORESIS DEVICE, AND ELECTROPHORESIS METHOD**

(30) Priority: 21.03.2013 JP 2013059107
(71) Applicant: NEC Corporation, Tokyo 108-8001 (JP)
(72) Inventor: ASOGAWA, Minoru, Tokyo 108-8001 (JP); HAGIWARA, Hisashi, Tokyo 108-8001 (JP); MISHINA, Yoshinori, Tokyo 108-8001 (JP); IIMURA, Yasuo, Tokyo 108-8001 (JP)
(74) Representative: Betten & Resch
(86) International application number: PCT/JP2014/054065
(87) International publication number: WO 2014/148193

(57) **Abstract**

An electrophoresis apparatus contributing to improving precision in analysis is provided. The electrophoresis apparatus comprises a membrane heater and controller. The membrane heater is supplied with power from a power supply and has a self-temperature control function. At the time of electrophoresis using a capillary extending inside of a microchip in a first direction, the controller controls supply of power from the power supply to the membrane heater arranged so as to supply heat to a capillary to keep the capillary at an even temperature.

## Description

### FIELD

### [Cross-Reference to Related Application]

The present Application claims benefits of priority of a Patent Application 2013-059107 filed in Japan on March 21, 2013, the entire contents thereof being incorporated by reference into the present application.

This invention relates to a method and an apparatus for electrophoresis and, more particularly, to a method and an apparatus for electrophoresis with a microchip in which a plurality of reaction chambers are communicated with fine flow paths.

### BACKGROUND

Electrophoresis is carried out for DNA (deoxyribonucleic acid), ions or low molecular compounds as targets for analysis. In particular, since individual identification with DNA is a useful means for efficiently narrowing down candidates in criminal investigation, there is an increasing need for electrophoresis for DNA as a target.

Patent Literatures 1 to 5 disclose microchips in which charging chambers and fine flow paths are arranged on a single chip. The microchips of Patent Literatures 1 to 5 have a multi-layered structure in which a plurality of plates are laminated, and sample chambers and reaction chambers are formed by perforations on a part of the plurality of plates. In addition, these sample chambers and reaction chambers are pressurized from outside to extrude the solution into the fine flow paths between the sample chambers and reaction chambers to control transfer of the solutions.

Furthermore, non Patent Literature 1 discloses a DNA analysis apparatus that carries out process steps necessary for DNA analysis on a microchip.

### CITATIONS LIST

### PATENT LITERATURE

PATENT LITERATURE 1: International Application Publication No. WO2008/108481A
PATENT LITERATURE 2: International Application Publication No. WO2009/035061A
PATENT LITERATURE 3: International Application Publication No. WO2009/035062A
PATENT LITERATURE 4: International Application Publication No. WO2009/038203A
PATENT LITERATURE 5: International Application Publication No. WO2009/119698A

### NON-PATENT LITERATURE

Non-Patent Literature 1: NEC Corporation, "Individual Identification with DNA and Technology thereof", September 2010, [online], [Retrieved on January 23, 2013, Internet <URL:http://www.nec.co.jp/techrep/ja/journal/g10/n03/100307.pdf>

### SUMMARY

### TECHNICAL PROBLEM

The disclosures of the above mentioned related technical literatures are to be incorporated herein by reference. The following analysis is made by the present inventors.

Individual identification with DNA is utilized in criminal investigation, as noted above. There is a demand that a sample, taken at a crime scene, is collected to carry out individual identification at that scene. To meet such demand, certain limitations are imposed on the size as well as weight of the DNA analysis apparatus. If, for example, the size of the DNA analysis apparatus is that of a small refrigerator, it would be extremely difficult to carry it to the crime scene.

Thus, it may be envisioned that the process steps necessary for DNA analysis are carried out on a microchip as disclosed in Non-Patent Literature 1. That is, the process steps necessary for the DNA analysis, such as DNA extraction, DNA amplification or decision of DNA length by electrophoresis are carried out on a microchip so as to realize downsizing of the DNA analysis apparatus.

However, the results of our perseverant researches have revealed that there is room for improvement in the DNA analysis apparatus disclosed in Non-Patent Literature 1. That is, when the DNA analysis apparatus is used at the crime scene and the like, the apparatus is preferably smaller in size. On the other hand, if the fact that the DNA analysis apparatus is used on the crime scene and the like is taken into account, it is necessary that the DNA analysis has high analysis accuracy.

If each site of a capillary used for electrophoresis is not maintained at a preset temperature, the speed of migration of DNA or the like target differs from one site of the capillary to another, with the result that a DNA length cannot be measured precisely. Additionally, even if each site of a capillary used for electrophoresis be maintained at a preset temperature, but the temperature at the capillary varies with lapse of time, electrophoresis may be affected in reproducibility. In short, if, in conducting electrophoresis for DNA of the same length, the temperature at the capillary varies with lapse of time, it may not be expected to realize high reproducibility.

On the other hand, in conducting electrophoresis, the temperature at the capillary is usually set at a higher temperature than the room temperature. For this reason, it is necessary to use a heater to warm ambient area of the capillary to maintain an even (or constant) temperature. However, in connection with this point, there is no disclosure in Non-Patent Literature 1 as to which means is to be used to maintain the capillary at an even temperature.

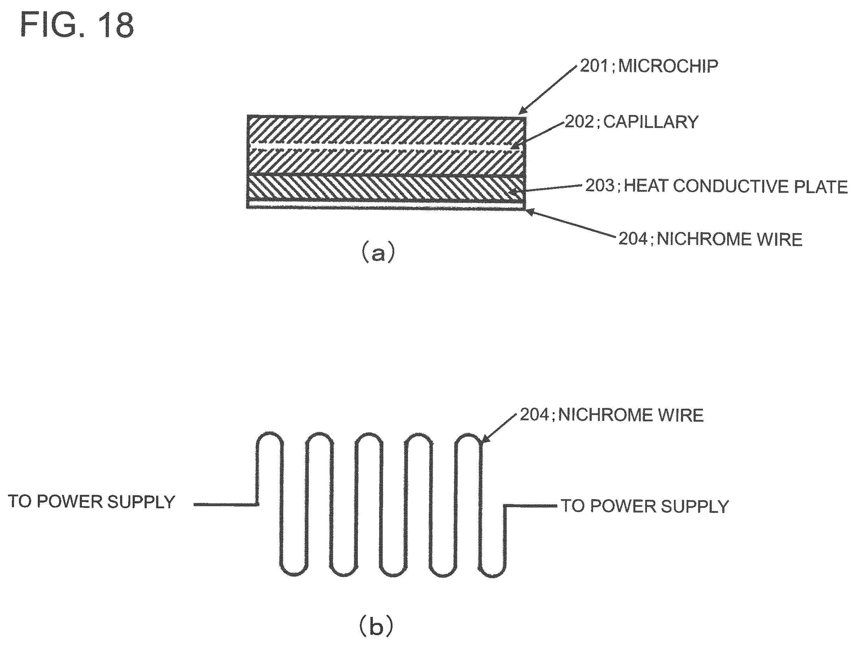

For example, a heat conductive plate 203, mainly made of aluminum having a high thermal conductivity, is contacted with a lower part of a microchip 201, provided internally with a capillary 202, as shown in Fig.18. It may be contemplated to provide a nichrome wire 204 on the lower part of the heat conductive plate 203, as shown in Fig.18(a), in order to control the temperature at the capillary 202. The nichrome wire 204 is provided in a bellows-like manner in order to maintain an even temperature at the capillary 202. See Fig.18(b) which is a plan view of the nichrome wire 204 placed on the lower part of the microchip 201. In short, by supplying power to the nichrome wire 204 laid in the bellows-like manner, heat may be supplied from the nichrome wire 204 to the heat conductive plate 203. The so supplied heat is diffused by the heat conductive plate 203 to maintain an even temperature on the surface of the microchip 201 contacting the heat conductive plate 203.

However, such configuration tends to generate temperature unevenness on the heat conductive plate 203 in a similar manner to the arrangement of the nichrome wire 204. In short, even if the material having s high thermal conductivity is selected as a main material of the heat conductive plate 203, there is a certain limit to the performance of maintaining an even temperature of the heat conductive plate 203, such that it is difficult to maintain an even over the major part of the surface of the heat conductive plate contacted with the microchip 201. Such temperature unevenness is ascribable to difference in generation and dissipation of heat at different sites of the heat conductive plate 203. Since the heat source of heat supplied to the heat conductive plate 203 is the nichrome wire 204, a site contacting the nichrome wire 204 is high in temperature. It is the role of the heat conductive plate 203 to moderate temperature rising at the site directly contacting to the nichrome wire 204. On the other hand, heat dissipation occurs at a peripheral part of the heat conductive plate 203, so that the temperature of an outer peripheral part of the heat conductive plate 203 is lowered. If the outer peripheral part of the heat conductive plate 203 is lowered in temperature, an outer peripheral part of the microchip 201 contacting the heat conductive plate 203 is also lowered in temperature.

Alternatively, one surface of the microchip 201 is virtually finely divided and a temperature sensor as well as a heater is contacted with each divided part to individually control each divided part. However, this method is unrealistic because of complex control, costs incurred in temperature sensors and so forth. If the capillary has temperature unevenness at respective sites, the analysis accuracy in electrophoresis is affected, as stated above. However, it is the current status that there is no means for heating the capillary while suppressing unevenness in temperature.

It is an object of the present invention to provide a method and an apparatus for electrophoresis contributing to improvement in the analysis accuracy.

### SOLUTION TO PROBLEM

In a first aspect of the present invention, there is provided an electrophoresis apparatus comprising a membrane heater supplied with power from a power supply and having a self-temperature control function, and a controller that, at the time of electrophoresis with at least one capillary extending inside of a microchip in a first direction, controls the supply of power from the power supply to the membrane heater. The membrane heater is arranged so as to supply heat to the capillary to keep the capillary at an even temperature.

In a second aspect of the present invention, there is provided a method for electrophoresis making use of electrode chambers into which electrodes are introduced and at least one capillary connected to the electrode chambers, in which the method comprises the steps of applying a dc current via the electrode chambers to a target of analysis and supplying a power to a membrane heater which is arranged so as to supply heat to the capillary to keep the capillary at an even temperature. The heater has a self-temperature controlling function.

### ADVANTAGEOUS EFFECTS OF THE INVENTION

In these aspects of the present invention, there may be provided a method and an apparatus for electrophoresis contributing to improvement in the analysis accuracy.

### BRIEF DESCRIPTION OF THE DRAWINGS

Fig.1 is a schematic view for illustrating a summary of an exemplary embodiment.
Fig.2 is a perspective view showing a configuration of a DNA analysis apparatus 10 according to exemplary embodiment 1.
Fig.3 is a view showing an exemplary configuration of a microchip 100.
Fig.4 is an exemplary cross-sectional view taken along line A-A of Fig.3.
Fig.5 is a flowchart showing an exemplary PCR program.
Fig.6 is an exemplary plan view showing a region of a table 12 inclusive of a temperature adjustment unit 13.
Fig.7 is an exemplary cross-sectional view taken along line B-B of Fig.6.
Fig.8 is an exemplary plan view showing a region of the table 12 inclusive of a temperature adjustment unit 14.
Fig.9 is an exemplary plan view showing a region of the table 12 inclusive of an electrophoresis unit 15.
Fig.10 is an exemplary cross-sectional view taken along line C-C of Fig.9.
Fig.11 is an exemplary plan view of a PTC heater 153.
Fig.12 shows positional relationship between electrode interconnects 158a, 158b of the PTC heater 153 and a capillary 116 in the microchip 100.
Fig.13 is an exemplary plan view showing the PTC heater 153.
Fig.14 is another exemplary plan view of the PTC heater 153.
Fig.15 is a flowchart showing an exemplary operation of the DNA analysis apparatus 10.
Fig.16 shows an example of temperature distribution on the heat conductive plate 151.
Fig.17(a) and Fig.17(b) show exemplary analysis results obtained by electrophoresis.
Fig.18(a) and Fig.18(b) show exemplary configurations of an electrophoresis section of the microchip.
Fig.19 is an exemplary cross-sectional view of the microchip 100 and the PTC heater 153.
Fig.20 is another exemplary cross-sectional view of the microchip 100 and the PTC heater 153.

### MODES

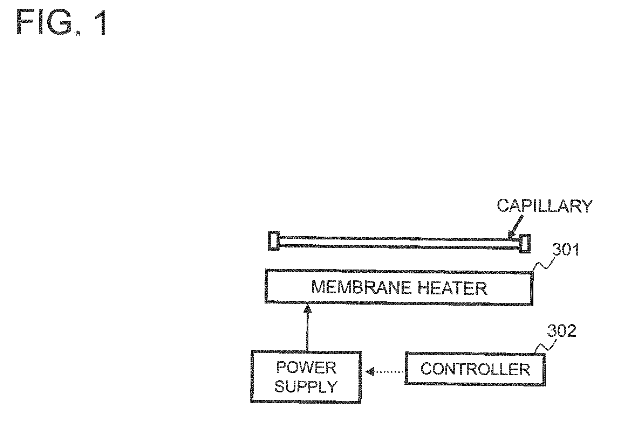

In first, a mode of the present invention will now be summarized with reference to Fig.1. It is noted that symbols in the summary are merely examples to assist in understanding and are not intended to limit the present invention to the mode shown in the summary.

An electrophoresis apparatus according to an exemplary embodiment comprises a membrane heater 301 supplied with power from a power supply and having a self-temperature control function; and a controller 302 that, at the time of electrophoresis using at least one capillary extending inside of a microchip in a first direction, controls the supply of (electric) power from a power supply to the membrane heater 301. The membrane heater 301 is arranged so as to supply heat to the capillary to keep the capillary at an even temperature (see Fig.1).

The membrane heater 301 has a self-temperature control function in which, when the own temperature of the membrane heater has reached a preset value, its electrical resistance is lowered to suppress heat generation. Thus, in case the microchip, including the membrane heater 301 and the capillary (or capillaries), is contacted with e.g., a heat conductive plate having a high thermal conductivity, the membrane heater 301 may supply each part of the capillary (or capillaries) with heat in an even manner. By evenly supplying heat to each part of the capillary, the ambient temperature of the capillary can be made substantially equal to improve precision in the electrophoresis analysis.

### An exemplary embodiment will now be explained specifically.

### [Exemplary Embodiment 1]

Exemplary Embodiment 1 will be explained in detail with reference to the drawings.

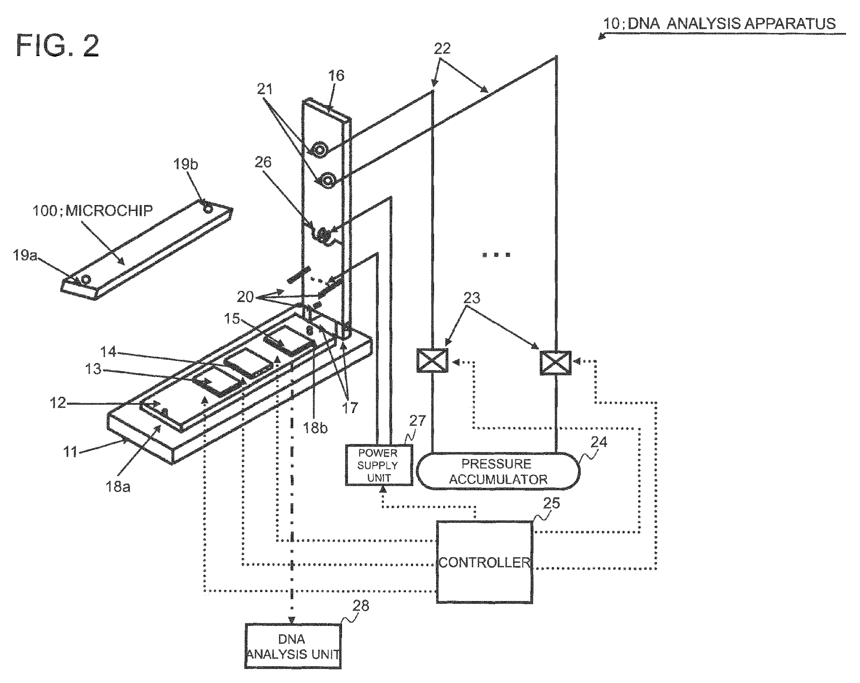

Fig.2 depicts a perspective view showing a configuration of a DNA analysis apparatus 10 according to the subject exemplary embodiment.

Referring to Fig.2, a table 12 is arranged on a base member 11. In the table 12, temperature adjustment units 13, 14 are embedded. The temperature adjustment unit 14 is also referred to as a temperature adjustment section. An electrophoresis unit 15 is arranged on the table 12. The base member 11 and a lid 16 are joined with a hinge 17 to allow opening/ closure of the lid 16.

A microchip 100 used in the DNA analysis apparatus 10 according to the subject exemplary embodiment is of a multi-layer structure in which a plurality of plates are laminated, as disclosed in Patent Literature 5. Sample chambers and reaction chambers are formed by perforations on a part of the plurality of plates. The microchip 100, used for DNA analysis, is installed at a predetermined position in a manner where pins 18a, 18b provided on the table 12 are engaged with pin holes 19a, 19b provided on the microchip 100. If, in a state that the microchip 100 is arranged on the table 12, the lid 16 is closed, certain regions of the microchip 100 are brought into contact with the temperature adjustment units 13, 14. In addition, by closing the lid 16, certain regions of the microchip 100 are brought into contact with a surface of the electrophoresis unit 15, at the same time as electrodes 20 are introduced into electrode chambers, respectively, provided on the microchip 100.

A plurality of pressurizing holes 21 are provided on the lid 16. The pressurizing holes 21 are through-holes formed on the lid 16 and are connected to solenoid valves 23 via tubes 22. On closing the lid 16, the pressurizing holes 21, formed on the lid 16, are brought into contact with certain regions on the microchip 100.

A pressure accumulator 24 stores compressed air which may be released via the pressurizing holes 21 on the lid 16 by the controller 25 controlling the solenoid valves 23. The internal pressure within the pressure accumulator 24 is controlled by a pressure sensor, a pump etc., not shown, so as to be maintained at a predetermined value of pressure. By the way, the microchip 100 of the subject exemplary embodiment has a flow path opening/ closing function disclosed e.g., in Patent Literature 5. The controller 25 controls the solenoid valve(s) 23 to apply pressure on a part of the microchip 100 via the pressurizing holes 21 formed on the lid 16. This extrudes the solution from a reaction chamber to a flow path provided on the microchip 100 so as to transfer the solution to an objective reaction chamber. For example, in a case where the solution is to be transferred from a reaction chamber A into a reaction chamber B, part of a flow path ahead of the reaction chamber B is pressured, while a flow path between the reaction chambers A, B is not. If the reaction chamber A is pressured under such state, the solution, accumulated in the reaction chamber A, is extruded into a flow path communicating the reaction chambers A and B. Since the flow path ahead of the reaction chamber B is pressured, the solution extruded is caused remains in the reaction chamber B. Transfer of the solution between the reaction chambers is achieved in such manner.

Moreover, an electromagnet coil 26 is arranged on the lid 16 and supplied with power from a power supply unit 27 so that a magnetic field may be generated in a predetermined region on the microchip 100. It is noted that the controller 25 instructs the power supply unit 27 to supply and to stop the supply of the electrical power to the electromagnet 26 so as to regulate excitation of the electromagnet 26.

The temperature adjustment units 13, 14 control the temperature of a predetermined region (or regions) on the microchip 100. The temperature adjustment units 13, 14 will be explained in detail below.

The electrodes 20 and the electrophoresis unit 15 are used in carrying out electrophoresis on the microchip 100. In more detail, in the process of the electrophoresis for the microchip 100, the controller 25 applies a dc voltage to the electrodes 20 via the power supply unit 27. When the dc voltage is applied to the electrodes 20, a charged DNA fragment migrates through the inside of the capillary. The electrophoresis unit 15 comprises a means for irradiating laser light and a means for receiving fluorescence emitted by excitation with the laser light irradiation. An output of the laser light receiving means, installed in the electrophoresis unit 15, is sent to a DNA analysis unit 28 so as to be used for analysis (determination) of the DNA length. By the way, the electrophoresis unit 15 will be explained in detail below.

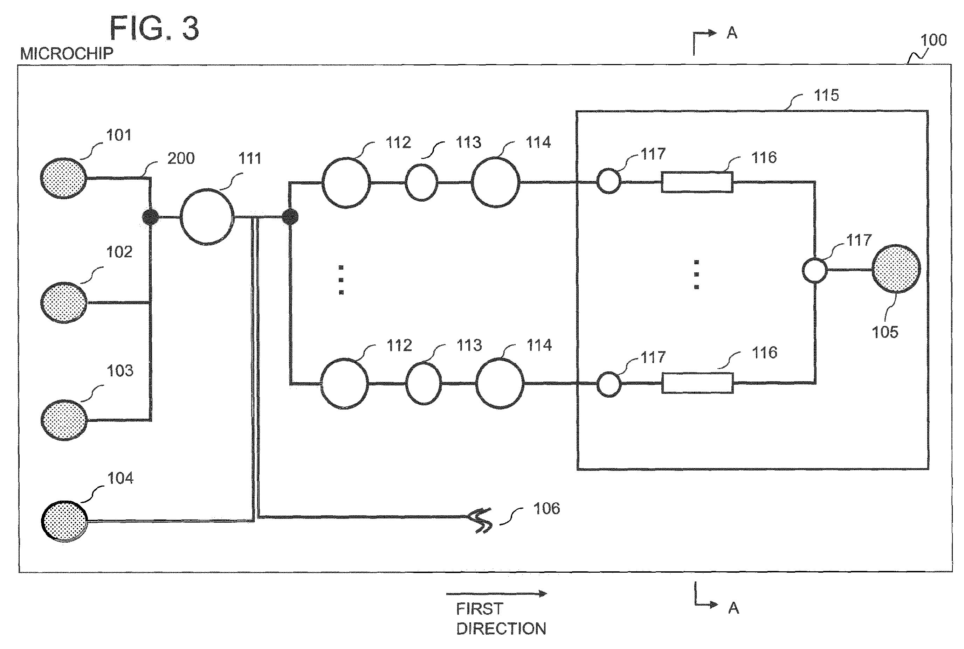

The configuration of the microchip 100 will now be explained. Fig.3 depicts an exemplary configuration of the microchip 100.

### [Configuration of a microchip]

Referring to Fig.3, the microchip 100 comprises a sample solution injection section 101, a wash buffer injection section 102, a PCR reagent injection section 103, a formamide injection section 104, an electrophoresis polymer injection section 105, a drainage port 106, a DNA extraction section 111, a PCR section 112, a volume determination section 113, a denaturing section 114, an electrophoresis section 115, a set of capillaries 116 and a flow paths 200 communicating the above sections etc.

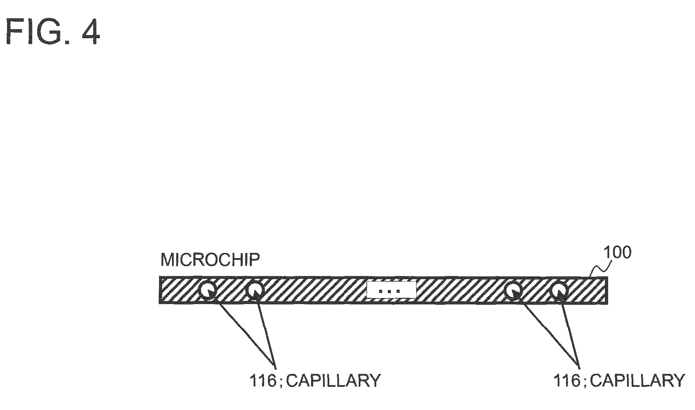

Each capillary 116 is provided inside of the microchip 100 and may be extended in a first direction shown in Fig.3. Fig.4 shows an exemplary cross-section taken along line A-A of Fig.3. A plurality of capillaries 116 are extended inside of the microchip 100. The microchip 100 is planar in shape and has a thickness smaller than its width or depth. The electrophoresis section 115 comprises a set of electrode chambers 117 into which a set of electrodes 20 arranged on the lid 16 is introduced at the time of DNA analysis. The set of electrode chambers 117 comprises an electrode chamber into which an anode (positive electrode) is introduced and an electrode chamber into which a cathode (negative electrode) is introduced. Both of these two types of the electrode chambers are connected to the capillaries 116.

The sample solution injection section 101 has a recessed structure into which a user injects a sample solution. The sample solution is a solution in which cells taken from a person, such as mouth mucosa, blood or body fluid, are suspended in a lysis buffer, such as SDS/LiOAc solution (sodium dodecyl sulfate/ lithium acetate solution).

The wash buffer injection section 102 also has a recessed structure into which a wash buffer is injected by the user. The wash buffer is e.g., a Tris buffer and being prepared at a high salt concentration to maintain binding of DNA to silica.

The PCR reagent injection section 103 also has a recessed structure into which a PCR reagent solution is injected by the user. A PCR reagent contains polymerase, dNTPs, magnesium and so forth and plays a role as an elution buffer for eluting DNA from silica. Hence, the PCR reagent is prepared at a low salt concentration.

The formamide injection section 104 also has a recessed structure and a formamide solution is injected by a user into this formamide injection section. A formamide solution is a reagent that keeps the DNA in a single-strand state. That is, repeated under denaturing process are denaturalization, also referred to as dissolving or separation, in which DNA is denatured from the double-strand state to the single-strand state and hybridization, also referred to as annealing or binding, in which DNA is converted from the single-strand state into the double-strand state. It should be noted that, formamide keeps DNA in the single-strand state, resulting in that formamide acts to denature the double-strand DNA to the single-strand DNA. As above, in the present Application, the terms 'keep' and 'denaturing' are sometimes used interchangeably. The formamide solution also contains an ssDNA (single-strand DNA) size marker labeled with a fluorescent dye.

The electrophoresis polymer injection section 105 also has a recessed structure, into which a polymer for electrophoresis is injected by the user.

By the way, the lysis buffer, wash buffer, PCR reagent, formamide, ssDNA size marker as well as the polymer are commercially available. These reagents may also be prepared in a different composition. The wash buffer, PCR reagent, formamide solution as well as the polymer may be pre-sealed in the microchip 100 instead of being injected by the user.

The DNA extraction section 111 is a reaction chamber to extract DNA from the sample solution. In the following description, the DNA extracted from the sample solution is also referred to as a template DNA.

The processing of extracting DNA will now be explained in detail. The DNA analysis apparatus 10 comprises an electromagnet 26 facing the DNA extraction section 111. In this DNA extraction section 111, silica-coated magnetic beads are pre-sealed. In the DNA analysis apparatus 10, the sample solution injected into the sample solution injection section 101 is transferred to the DNA extraction section 111 where the sample DNA is adsorbed to the magnetic beads (silica) sealed in the DNA extraction section 111. The magnetic beads are rinsed with the wash buffer in the wash buffer injection section 102 to extract the template DNA. It should be noted that, when the DNA analysis apparatus 10 discharges the sample solution and the wash buffer via the drainage port 106, the magnetic beads are absorbed by the electromagnet 26 to prevent the magnetic beads from being discharged along with the sample solution and the wash buffer.

By the way, as a method for DNA extraction with the magnetic beads, it is known to use a MagExtractor (registered trademark) manufactured by TOYOBO CO. LTD and NucleoMag (registered trademark) manufactured by TAKARA-BIO CO. LTD. The protocol for DNA extraction may be modified as necessary such as by increasing the number of times of rinsing. The method for DNA extraction is not limited to using the magnetic beads. A silica beads column, for example, may be used to extract the template DNA. See for example QIAamp of Qiagen Co. Ltd.

The PCR section 112 is one or more reaction chambers provided halfway in order to carry out PCR which amplifies a desired segment of the template DNA. Each PCR section 112 is provided adjacent to the temperature adjustment unit 13. In each PCR section 112, there is sealed a primer set designed to amplify a desired segment of the template DNA.

The primer set is a forward primer and a reverse primer for PCR amplification of a segment comprising a microsatellite, such as TPOX or FGA. One or both of the primers are labeled with fluorescent dye, such as fluorescein. Such primer is commercially available from Promega Corporation (Promega, a registered trademark), and may also be designed as necessary. A plurality of primer sets may be sealed in one PCR section 112.

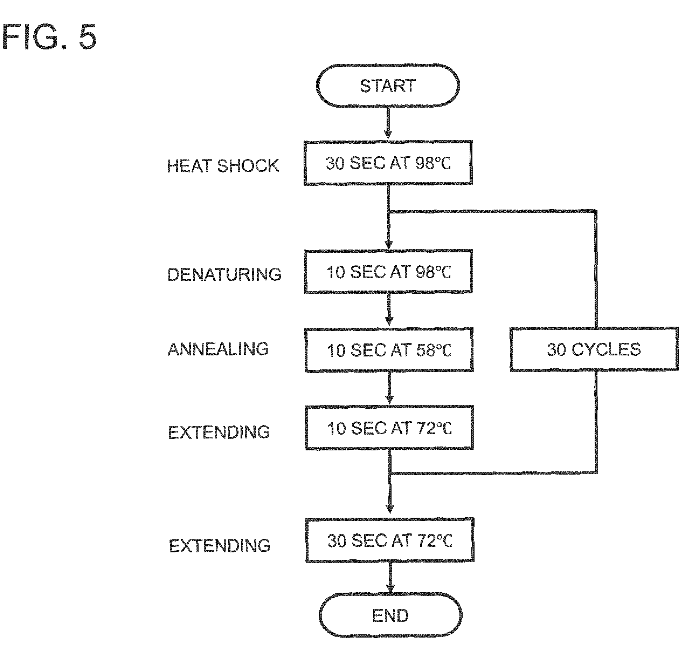

The PCR (the polymerase chain reaction) will now be specifically explained. In the DNA analysis apparatus 10, a PCR reagent containing a template DNA is transferred from the DNA extraction section 111 to a plurality of the PCR sections 112. The temperature of the PCR sections 112 is controlled in a programmed manner by a heat conductive material of the temperature adjustment unit 13. In an example PCR programming, the DNA analysis apparatus 10 executes PCR by temperature control in accordance with temperature and time setting shown in Fig.5. The temperature conditions of the PCR as well as the number of cycles may be modified based on the Tm (melting temperature) value or the length of the amplicon. The DNA, amplified by PCR, is referred to as 'amplicon' and a PCR reagent containing the amplicon is referred to as a reaction sample hereinafter.

The volume determination section 113 is a reaction chamber for disposing a part of the reaction sample, particularly having a smaller capacity than the PCR section 112. The volume determination processing will now be explained. The DNA analysis apparatus 10 transfers the reaction sample from the PCR section 112 to the volume determination section 113 until the volume determination section 113 is filled up, while discharging the remaining reagent via the drainage port 106.

The denaturing section 114 is a reaction chamber for denaturing the amplicon from the double strand DNA (dsDNA) to the single stand DNA (ssDNA), and is arranged adjacent to the temperature adjustment unit 14. The denaturing processing will now be explained in detail. The temperature of the denaturing section 114 is kept by the DNA analysis apparatus 10 at a preset temperature, such as 60°C, via the temperature adjustment unit 14. The DNA analysis apparatus 10 transfers formamide injected into the formamide injection section 104 to the denaturing section 114 via the PCR section 112 and the volume determination section 113. Since the amplicon amplified in the PCR section 112 flows into the denaturing section 114 together with the keeping reagent (formamide), the amplicon and the keeping reagent may be mixed together more hardly when compared with a case where the amplicon and the keeping reagent are allowed to flow independently into the denaturing section 114. The DNA analysis apparatus 10 operates to hold the reaction sample in the denaturing section 114 for a preset reaction time.

The electrophoresis section 115 is configured to separate the amplicon in accordance with the length of nucleic acid sequence by the molecular sieve effect, and is arranged so as to be adjacent to the PTC heater which will be explained below. More specifically, the electrophoresis section 115 comprises the capillaries 116 and is arranged adjacent to the PTC heater so as to maintain the capillaries 116 at an even temperature.

The processing of electrophoresis will be explained in more detail. The polymer in the electrophoresis polymer injection section 105 is charged into the capillaries 116 by the DNA analysis apparatus 10. The electrophoresis section 115 is maintained by the PTC heater at a preset temperature, such as at 50°C. The DNA analysis apparatus 10 transfers the reaction sample from the denaturing section 114 to the electrophoresis section 115 to inject the reaction sample into each of the capillaries 116. As an injection method, a so-called cross-injection method may be used (see for example JP Patent Publication No.2002-310858A). When starting peak detection by a light receiving means, the DNA analysis apparatus 10 applies a dc voltage via the electrode chambers 117 connected to the capillaries 116.

The temperature adjustment units 13, 14 will now be explained.

The temperature adjustment unit 13 is a means for controlling temperature of the PCR section 112 on the microchip 100 under instructions from the controller 25.

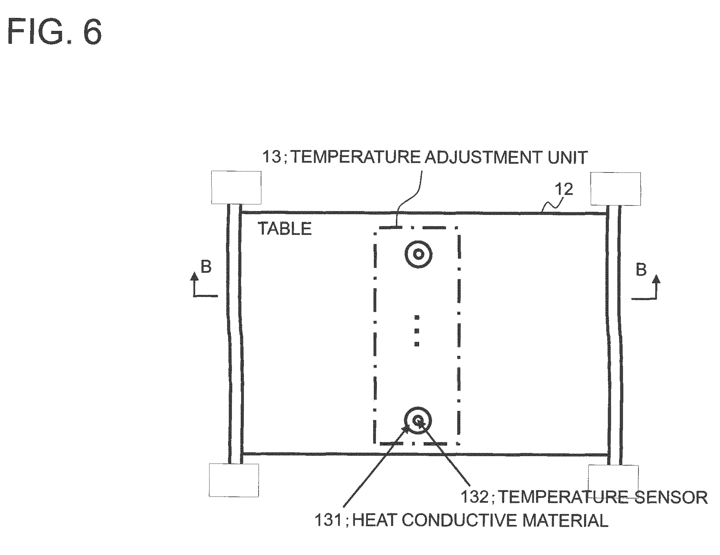

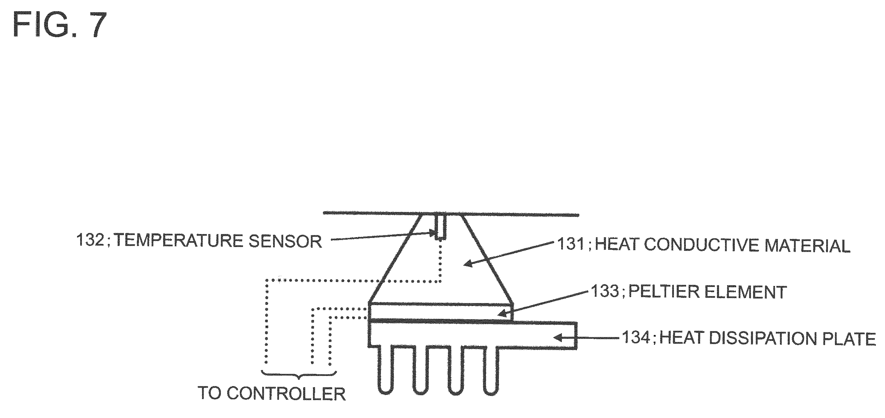

Fig.6 depicts an exemplary plan view showing a certain region of the table 12 comprising the temperature adjustment unit 13. Fig.7 depicts an exemplary cross-sectional view taken along line B-B of Fig.6.

The temperature adjustment unit 13 is arranged in a certain region of the table 12 in an embedded manner, as stated above. Referring to Fig.6, a heat conductive material 131 is exposed on a surface of the table 12, and a temperature sensor 132 is arranged at the center of the heat conductive material 131.

Referring to Fig.7, the temperature sensor 132 is connected to the controller 25. The heat conductive material 131 has its one surface in contact with a temperature sensing (temperature applying) surface of a Peltier element 133. The Peltier element 133 has its temperature dissipation surface in contact with a surface of a heat dissipation plate 134. A power supply line of the Peltier element 133 is connected to the controller 25. The controller 25 acquires the temperature of the PCR section 112 from the temperature sensor 132 and, based on the so acquired temperature, decides the direction of the current delivered to the Peltier element 133 to control the heating or cooling of the Peltier element 133 to manage temperature control of the PCR section 112.

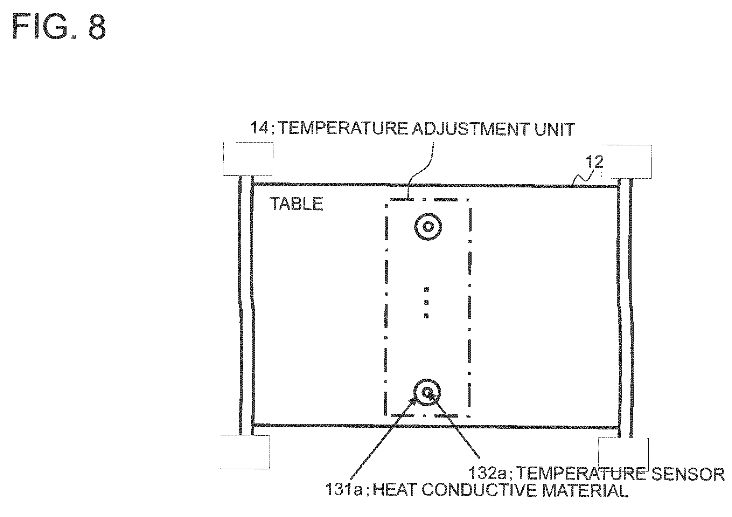

The temperature adjustment unit 14 is a means for maintaining an even temperature of the denaturing section 114 on the microchip 100 based on an instruction from the controller 25. Fig.8 depicts an exemplary plan view showing a region of the table 12 including the temperature adjustment unit 14. The temperature adjustment unit 14 may have the same configuration as the temperature adjustment unit 13, as shown in Fig.8. It is however not intended to limit the structure of the temperature adjustment unit 14, such that it is possible to construct the temperature adjustment unit 14 using a heater, as an example.

The electrophoresis unit 15 will now be explained.

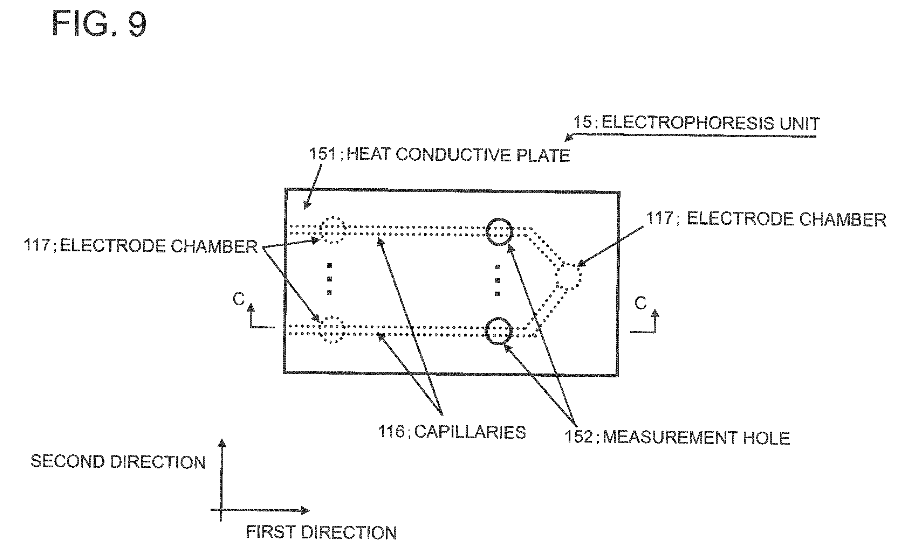

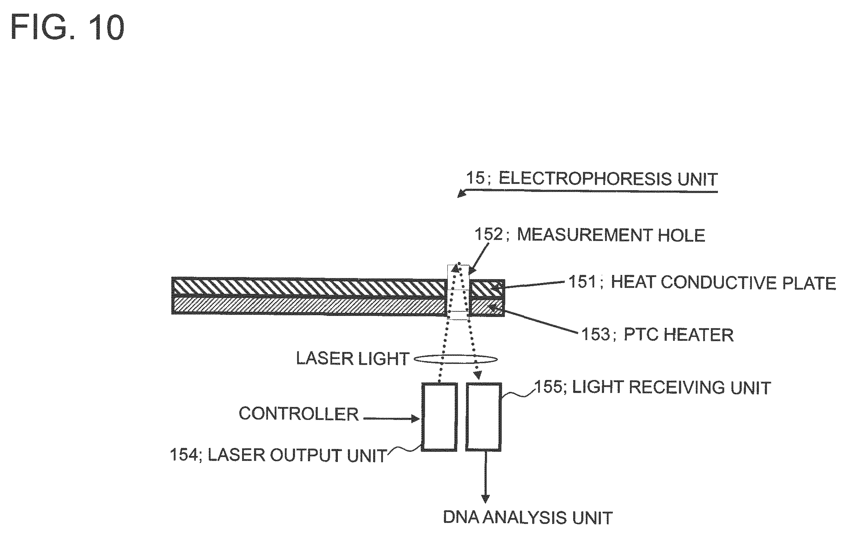

Fig.9 depicts an exemplary plan view showing a region of the table 12 comprising the electrophoresis unit 15. Fig.10 depicts an exemplary cross-sectional view taken along line C-C of Fig.9. It is noted that the capillaries 116, shown by dotted line in Fig.9, are provided not in the electrophoresis unit 15 but inside of the microchip 100. The set of electrode chambers 117, which are shown by dotted circles in Fig.9, and into which the electrodes 20 are introduced, are also provided in the microchip 100. These components are only shown to assist in understanding in Fig.9

Referring to Fig.9, the electrophoresis unit 15 comprises a heat conductive plate 151, a through-hole 152 for passage of laser light used for measuring the DNA length is arranged thereon. Referring to Fig.10, a PTC (positive temperature coefficient) heater 153 is arranged on the bottom surface of the heat conductive plate 151 in a laminated manner on the heat conductive plate 151. The measurement hole 152 is also provided on the PTC heater 153.

A laser output unit 154 comprises a laser diode that emits laser light towards the measurement hole 152. On/off of the irradiation of the laser light by the laser output unit 154 is controlled in accordance with instructions from the controller 25. A light receiving unit 155 receives fluorescence emitted from the DNA fragments passing through a site corresponding to the measurement hole 152 provided on the electrophoresis section 115. The light receiving unit 155 comprises a photomultiplier, as an example. The light receiving unit 155 converts the light reflected by a DNA, which has migrated by electrophoresis through the capillaries to a site directly above the measurement hole 152, into an electrical signal, which is output to the DNA analysis unit 28. Or, the light receiving unit 155 may comprise an image pickup element, such as a charge-coupled device (CCD), measuring the intensity of the reflected light, so as to detect the passage of the DNA through a site directly above the measurement hole 152.

It should be noted that the electrophoresis unit 15 radiates the laser light from below the microchip 100, that is, in a direction proceeding from the table 12 of Fig.2 towards the lid 16. However, laser light radiation from the electrophoresis unit 15 is not limited to radiation from below the microchip 100. If, for example, the electrophoresis unit 15 is attached on the lid 16, the laser light is radiated from above the microchip 100. In this case, there is no necessity of providing the measurement hole 152 on the heat conductive plate 151.

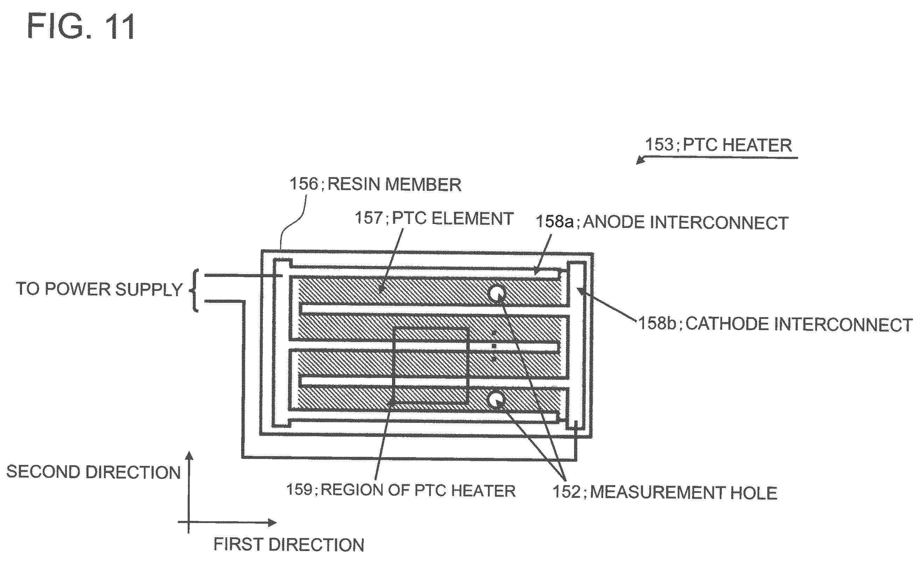

Fig.11 depicts an exemplary plan view of the PTC heater 153. The PTC heater 153 is a membrane heater comprising a PTC element 157, an anode interconnect 158a and a cathode interconnect 158b on a resin member 156 having the same shape as the heat conductive plate 151. The PTC heater 153 is arranged for supplying heat to the capillaries 116 and maintaining the capillaries 116 at an even temperature. The resin member 156 is of a size to fit to the electrophoresis section 115 of the microchip 100, thus of a size substantially equal to the electrophoresis section 115. The PTC element 157 as well as the electrode interconnects 158a, 158b are arranged on a surface of the resin member 156 which is in contact with the heat conductive plate 151. On applying a dc voltage across the electrode interconnects 158a, 158b, current flows through the PTC element 157. When the current flows through the PTC element 157, heat is generated by the PTC element 157 and supplied via the heat conductive plate 151 to the capillaries 116.

It should be noted that the PTC element 157 has a feature that, in case current flows through it so that it has reached a preset temperature, its electrical resistance decreases acutely. That is, the PTC element 157 acts as a current limiting element having a feature that when the current flows through the PTC element 157, its electrical resistance increases by self-heat generation to render current conduction difficult. If the current flowing through the PTC element 157 is decreased, power usage by the PTC element 157 is also decreased, resulting in that the temperature due to heat generation is lowered. The PTC heater 153, with the PTC element 157, thus has the self-temperature control function for maintaining a preset temperature. The electrode interconnects 158a, 158b of the PTC heater 153 are connected to the power supply unit 27. The controller 25 controls the operation of the PTC heater 153 via the power supply unit 27. When desired to maintain the electrophoresis section 115 at a preset temperature with the use of the PTC heater 153, the controller 25 instructs the power supply unit 27 to supply power to the PTC heater 153. Since the PTC heater 153 has the self-temperature control function, the electrophoresis section 115 of the microchip 100 may be maintained at the preset temperature via the heat conductive plate 151.

The configuration of the PTC heater 153 will now be explained.

The PTC element 157 and the electrode interconnects 158a, 158b are arranged on a surface of the resin member 156 which is in contact with the heat conductive plate 151. More specifically, the anode interconnect 158a, the positive voltage is applied to, and the cathode interconnect 158b, the grounding voltage is applied to, are arrayed in the first direction (longitudinal direction) along which the capillaries 116 extend inside of the microchip 100. The electrode interconnects are alternately arrayed on the resin member.

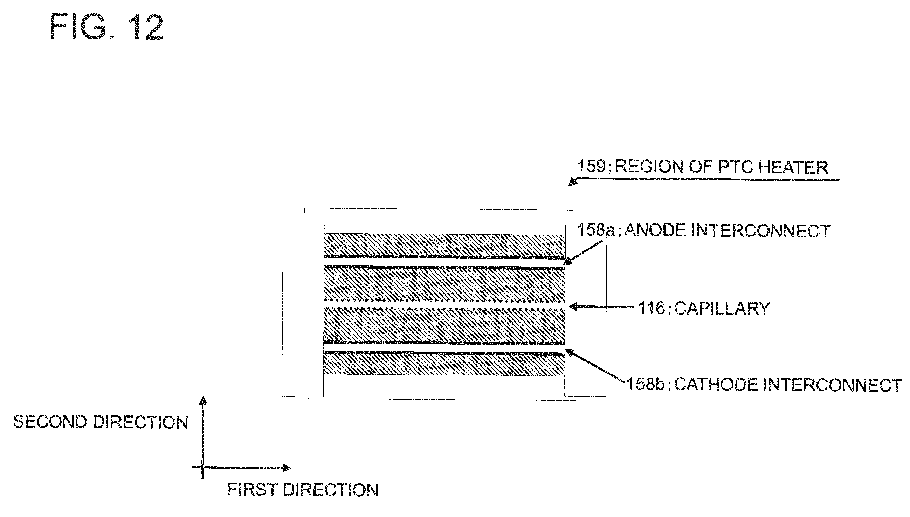

Fig.12 illustrates the position relationship between the electrode interconnects 158a, 158b of the PTC heater 153 and the capillaries 116 inside of the microchip 100. Fig.12 shows a region 159 of Fig.11 in an enlarged scale. In Fig.12, the capillaries 116 are shown with dotted lines. By referring to Fig.12, it is seen that the anode interconnect 158a and the cathode interconnect 158b are so arrayed that the capillary 116 is disposed on a middle section (part) between these anode and cathode interconnects. By arranging the anode interconnect 158a and the cathode interconnect 158b on both sides of the capillary 116 in this manner, the temperature at the capillary 116, disposed at a higher height, may be made even. The PTC element 157 has the self-temperature control function, as discussed above. Moreover, since the PTC element 157 is arranged on entire region between the electrode interconnects 158a, 158b disposed on both sides of the capillary 116, the temperature on the region may be regarded to be substantially the same. The reason is that, if temperature of the region surrounding the capillary 116 is the same, the heat supplied via the heat conductive plate 151 to the capillary 116 may be regarded to be the same. That is, it is possible to cancel unevenness in the temperature of the capillary 116 in the first direction.

The heat conductive plate 151 and the PTC heater 153 are provided with the measurement hole 152. In this case, heat diffusion takes place around the measurement hole 152. Thus, if a nichrome wire 204 described above is used as a heat source, instead of using the PTC heater 153 as the heat source, there is raised a problem that the temperature around the measurement hole 152 becomes lower than that in other sites. However, if the PTC heater 153 is used as the heat source, the self-temperature control function of the PTC element 157 comes into play, such that, even if the temperature around the measurement hole 152 is lowered owing to the very presence of the measurement hole, such temperature lowering may be compensated. That is, it is possible to render the temperature of the region around the measurement hole 152 and that in the other regions substantially equal to each other to remove the risk of temperature unevenness in the capillaries 116.

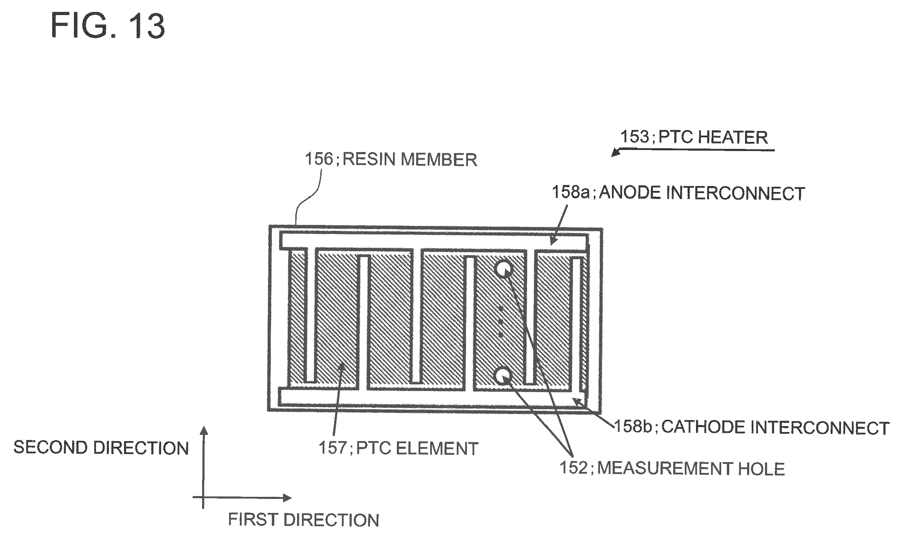

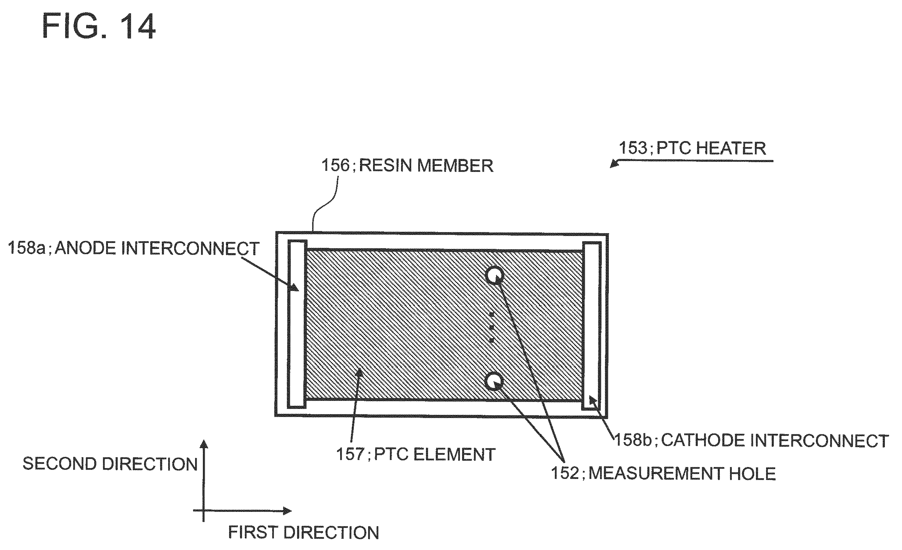

By the way, the manner of arraying the electrode interconnects 158a, 158b is not limited to that shown in Fig.11. For example, the pair electrode interconnects 158a, 158b may be arranged in the second direction (transverse direction), as shown in Fig.13. Or the pair electrode interconnects 158a, 158b may be arranged on both ends of the resin member 156, as shown in Fig.14, with the electrode interconnects 158a, 158b not being arranged in the center portion of the resin member 156.

In light of the purpose of providing an even temperature at the capillaries, the arrangement of Fig.11 is not appreciably different from that of Fig.14. However, in the arrangement shown in Fig.14, it is necessary to apply a voltage higher than that shown in Fig.11 to the anode interconnect 158a. In the arrangement shown in Fig.11, in which the region where the PTC element 157 is disposed is separated with the electrode interconnects 158a, 158b, it is possible to suppress the voltage applied to the anode interconnect 158a. Due to difference in the manner of laying out of the electrode interconnects 158a, 158b, the voltage applied to the PTC heater 153 differs in the two arrangements.

The arrangement shown in Fig.13 also differs from that shown in Fig.11 in that, when the PTC heater 153 is viewed from above, the capillary 116 crosses the electrode interconnects 158a, 158b. Since the PTC element 157 is not provided in regions of crossing of the capillary 116 and the electrode interconnects 158a, 158b, heat is not supplied to the regions. Hence, temperature unevenness is generated between these regions and neighboring regions, thus leading to a possibility that the temperature of the capillaries 116 may not be made even. However, in the arrangement of Fig.11, it is necessary to design the shape of the microchip 100 as well as the PTC heater 153 so that the capillaries 116 will not be overlapped with the electrode interconnects 158a, 158b, that is, so that the capillaries 116 will be disposed intermediate between the electrode interconnects 158a, 158b. On the other hand, with the arrangement shown in Fig.13, it is not strictly necessary to pay attention in designing the shape of the microchip 100 or the PTC heater 153. Moreover, if, in the arrangement shown in Fig.13, the electrode interconnects 158a, 158b extending in the second direction are reduced in width, the adverse effect on the temperature distribution of the capillaries 116 may be thought to be negligibly small. Thus, the manner of arranging the PTC element 157 and the manner or arraying the electrode interconnects 158a, 158b have merits and demerits. In light of the above, the manner of arraying the PTC element 157 and the electrode interconnects 158a, 158b is desirably decided as the power supply unit supplying the power to the PTC element 157, ease in designing and so forth are comprehensibly taken into account.

The operations by a user in carrying out DNA analysis as well as the operation of the DNA analysis apparatus will now be explained.

### [Operation by a user]

A user fills the sample solution injection section 101, wash buffer injection section 102, PCR reagent injection section 103, formamide injection section 104 and the electrophoresis polymer injection section 105 with respective solutions and sets the microchip 100 on the DNA analysis apparatus 10. The user then actuates the DNA analysis apparatus 10 to start DNA analysis.

### [Sequence of operations by the DNA analysis apparatus]

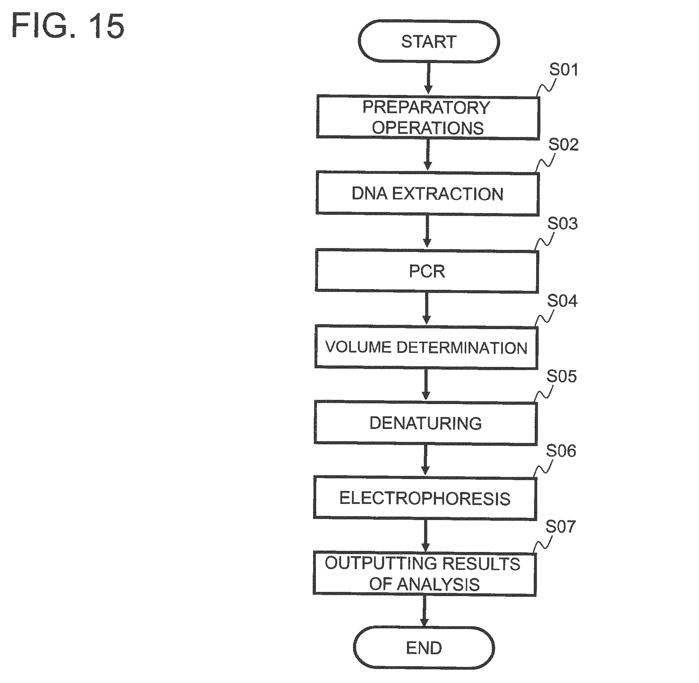

Fig.15 depicts a flowchart showing an example operation of the DNA analysis apparatus 10. When the user has set the microchip 100, and a command to initiate the processing has been admitted, the DNA analysis apparatus 10 carries out preparatory operations (step S01). More specifically, the DNA analysis apparatus 10 maintains the temperature of the denaturing section 114 at a preset value, such as 60°C, with the temperature adjustment unit 13, while maintaining the electrophoresis section 115, in particular the capillaries 116, at another preset value, such as 50°C, with the electrophoresis section 115. The DNA analysis apparatus 10 charges the polymer in the electrophoresis polymer injection section 105 into the capillaries 116.

The DNA analysis apparatus 10 then executes the processing of DNA extraction (step S02). More specifically, the DNA analysis apparatus 10 transfers the sample solution injected into the sample solution injection section 101 to the DNA extraction section 111 to cause the sample DNA to be adsorbed to the magnetic beads (silica) sealed in the DNA extraction section 111. The magnetic beads are rinsed with a wash buffer within the wash buffer injection section 102 to extract the template DNA. The DNA analysis apparatus 10 then transfers the PCR reagent injected into the PCR reagent injection section 103 to the DNA extraction section 111 to elute the sample DNA.

The DNA analysis apparatus 10 then carries out PCR (step S03). Specifically, the DNA analysis apparatus 10 transfers the PCR reagent containing the template DNA from the DNA extraction section 111 to a plurality of PCR sections 112, and performs temperature control of the PCR sections 112, as programmed, via the heat conductive material 131 of the temperature adjustment unit 13.

After accomplishment of the PCR, the DNA analysis apparatus 10 executes volume determination (step S04). Specifically, the DNA analysis apparatus 10 transfers the amplicon containing PCR reagent, referred to as a reaction sample, from the PCR section 112 to the volume determination section 113 until the volume determination section 113 is filled up, then discharging the residual PCR reagent via the drainage port 106.

The DNA analysis apparatus 10 then executes the processing of denaturing (step S05). Specifically, the DNA analysis apparatus 10 transfers formamide injected into the formamide injection section 104 to the denaturing section 114 via the PCR section 112 and the volume determination section 113. Thereby, the reaction sample and formamide are transferred to the denaturing section 114 while being mixed together. The DNA analysis apparatus 10 executes the denaturing processing as the reaction sample is maintained in the volume determination section 113 for a preset reaction time.

The DNA analysis apparatus 10 then executes the processing of electrophoresis (step S06). Specifically, the DNA analysis apparatus 10 transfers the reaction sample from the denaturing section 114 to the electrophoresis section 115 to inject the reaction sample into each capillary 116. The DNA analysis apparatus 10 initiates peak detection by the light receiving unit 155 of the electrophoresis unit, then applying a dc voltage to the capillaries 116 to carry out the processing of electrophoresis.

Finally, the DNA analysis apparatus analyzes DNA length, using the DNA analysis unit 28, and outputs the result of analysis (step S07).

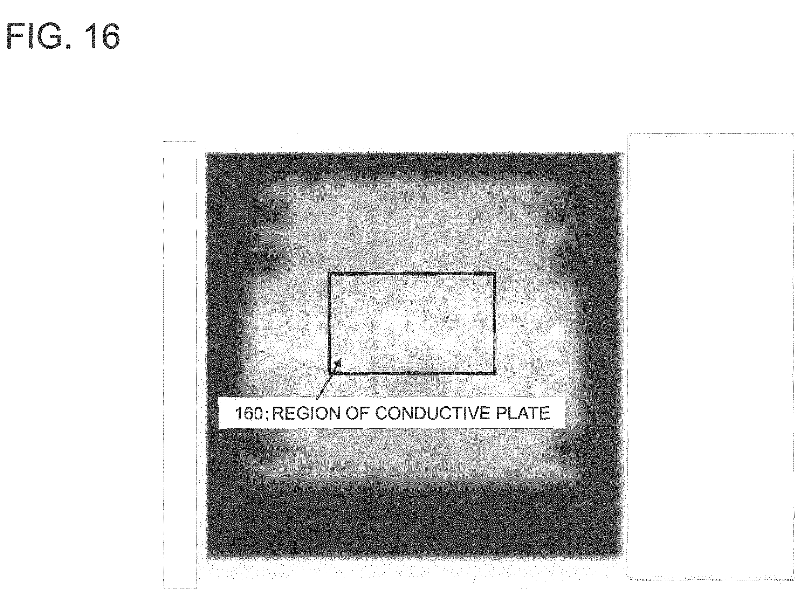

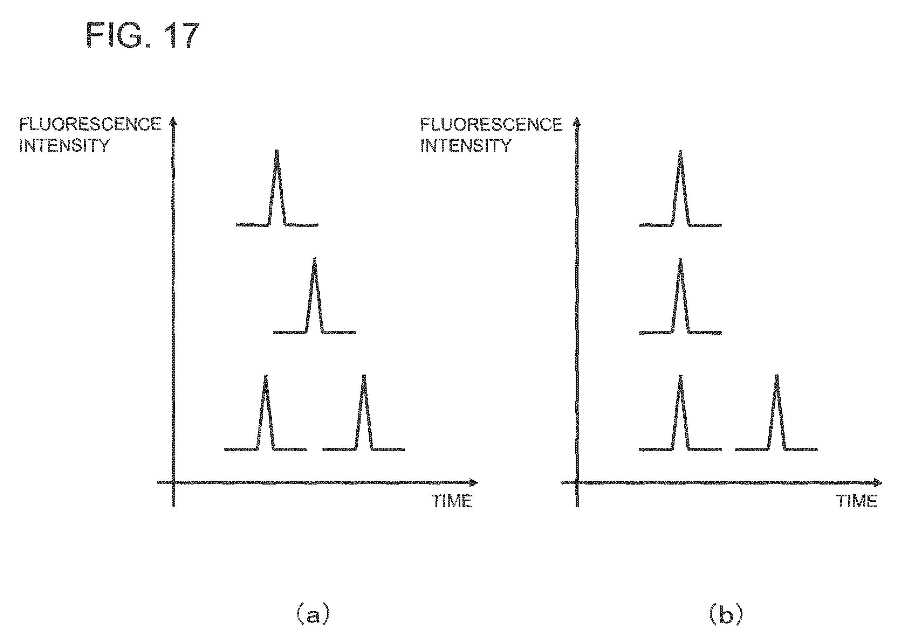

As described above, the DNA analysis apparatus 10 uses the PTC heater 153 to control the capillary temperature, as a result of which it is possible to suppress temperature unevenness on the heat conductive plate 151 contacting with the PTC heater 153. Fig.16 shows an example temperature distribution of the heat conductive plate 151. It is seen from Fig.16 that the temperature distribution in a region 160 that supplies heat to the capillaries 116 is substantially even. In Fig.16, relative color darkness represents the temperature level. If the temperature unevenness on the heat conductive plate 151 can be canceled, temperature unevenness on the capillaries 116 is also canceled, thus improving the precision in DNA analysis. That is, if the ambient temperature of the capillaries 116 is low, DNA migrates at a low speed, whereas, if the ambient temperature is high, DNA migrates at a high speed, thus providing a phenomenon known as smiling. If the peaks of the sample and the size marker are coincident at this time, but the smiling is generated, peaks detected are offset due to the difference in the migration speed (see Fig.17(a)). If conversely the ambient temperatures of the capillaries 116 are substantially the same, smiling can be suppressed, so that peak offsets due to the difference in the migration speed can be overcome (see Fig.17(b)).

In addition, in the DNA analysis apparatus 10, in which electrophoresis is carried out after the processing of denaturing, the analysis may be improved in precision. Since the amplicon contains a repeat sequence, the amplicon in the double strand state may likely have a cross-linked or bulge loop structure. Even if the amplicon is in the single strand state, it likely has a hairpin structure. These structures have different migration speeds from that of the liner single-strand amplicon, thus possibly providing ghost bands. In the exemplary embodiment 1, in which the processing of denaturing is performed, it is possible to eliminate the risk of the ghost bands, as a result of which the analysis may be improved in precision.

### [Modifications]

The above represents merely a preferred mode which may be modified in a number of ways. For example, the conditions for denaturing, such as temperatures, processing time, reagents or the volume of the solutions, may arbitrarily be modified. That is, a diversity of reaction conditions may be applied for denaturing the DNA. For example, the denaturing section 114 mixes the amplicon containing PCR reagent with a keeping reagent (formamide) at a mixing ratio of 1:2 to 1:9. A search conducted by the present inventors has revealed that sufficient results of the processing of denaturing may be obtained in case the mixing ratio of the reaction sample to formamide is 1:9 (1 *µ*l to 9 *µ*l) and the temperature is 60°C.

The temperature of the processing of denaturing is not limited to 60°C such that a temperature at which the amplicon as a double strand DNA is denatured to a single strand DNA is sufficient. That is, the temperature of the processing of denaturing is approximately 50 to 98°C depending on the sequence of the amplicon (Tm value) as well as the formamide/ reaction sample mixing ratio,

Although the time of the processing of denaturing, for example, is at least 30 sec, such time which is as long as that tolerable for the user is desirable.

The DNA denaturing agent is not limited to formamide, such that urea, for example, may be used.

The volume of the reaction sample, to be measured by determination process, i.e. the capacity of the volume determination section 113, is preferably as small as possible, provided that it is not so small as to adversely affect peak detection. That is, the greater mixing ratio of formamide to the reaction sample is applied, the higher efficiency of the denaturing would be provided, whereas the peak detected becomes smaller, thus modification should be made, if required. It has been confirmed that, with a sufficiently high denaturing temperature, sufficient results of denaturing may be obtained even if the mixing ratio of the reaction sample and formamide is 1:2.

In a manual operation, conducted at a laboratory, such a protocol is known in which an amplicon is purified by ethanol precipitation and dissolved in formamide. A sample containing the amplicon is heated to 98°C and then rapidly cooled to 0°C. This protocol may be referred to in order to provide an amplicon purifying configuration on the DNA analysis apparatus 10 and on the microchip 100. The amplicon, may, for example, be purified using the process of DNA extraction with the above mentioned magnetic beads. It is also possible for the DNA analysis apparatus 10 to perform temperature control so that the denaturing section 114 will be kept at 98°C and then rapidly cooled to 0°C. It is also possible for the temperature adjustment unit 14 by itself to manage temperature control. Or, a hollow structure as well as a temperature adjustment unit, configured for heating to 98°C, and a hollow structure as well as a temperature adjustment unit, configured for cooling to 0°C, may be provided independently of each other.

It is also possible for the PCR section 112 to execute the denaturing processing without carrying out the volume determination processing. For example, it is possible to add formamide after accomplishment of PCR and to manage temperature control for maintaining the PCR section 112 at 98°C and subsequently cooling it to 0°C. In such case, it is feared that, since the ratio of mixing the reaction sample to formamide becomes larger, the denaturing efficiency may tend to be lowered. However, it may be contemplated that, by adding the amplicon purifying process, it is possible to prevent the denaturing efficiency from being lowered. In such case, since the temperature adjustment unit 14, volume determination section 113 and the denaturing section 114 are not required, it may be expected to reduce the size of the DNA analysis apparatus 10.

On the other hand, it has been ascertained that, in case the mixing of the formamide and the reaction sample is insufficient, the efficiency of the denaturing processing is lowered. It is thus possible to add the processing of mixing of formamide and the reaction sample, such as shuffling the mixture solution between the PCR section 112 and the denaturing section 114.

The above described exemplary embodiment is directed to an electrophoresis apparatus used for DNA analysis. It is however not intended to limit the use of the electrophoresis apparatus to DNA analysis. For example, the subject of analysis may be ions or low molecular compounds. Additionally, DNA analysis is not limited to identification of individuals for criminal investigation and may also be used for detection of gene deletion.

### [Exemplary Embodiment 2]

An exemplary embodiment 2 will now be explained in detail with reference to the drawings.

In the exemplary embodiment 1, it has been stated that the PTC heater 153 has a membrane shape. However, in consideration of the function of the PTC heater 153 of supplying heat to the capillaries 116 and maintaining the even temperature, the overall shape of the PTC heater is obviously not limited to a membrane shape, i.e., it is only sufficient that only a part of the PTC heater has planar shape. That is, the PTC heater 153, explained in the exemplary embodiment 1, should encompass a heater of a three-dimensional shape, part of which is planar in shape, as a matter of interpretation. The subject exemplary embodiment is directed to the PTC heater 153 a part of which is planar in shape as stated above.

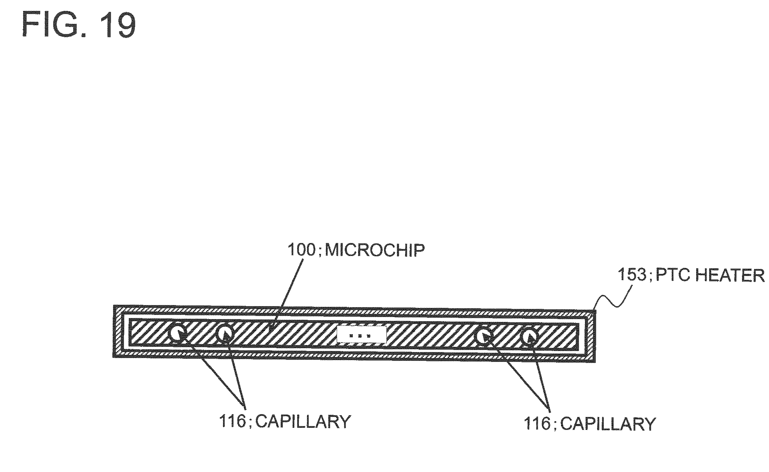

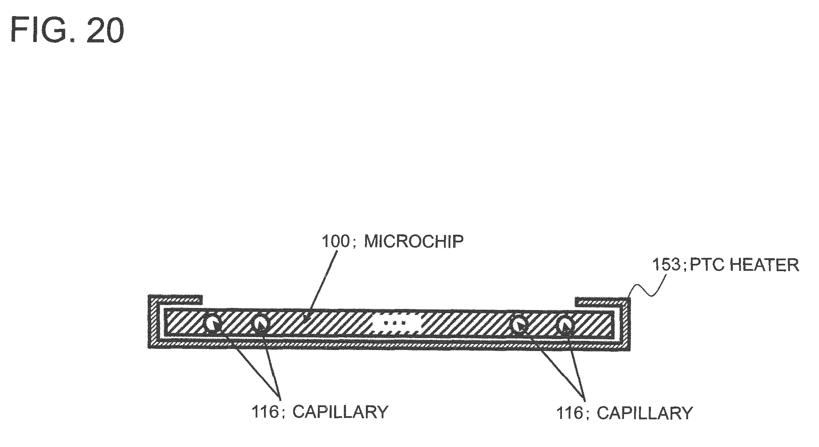

For example, the PTC heater 153 may be of a shape that encloses the microchip 100 in its entirety. Fig.19 depicts a cross-sectional view of the microchip 100 and the PTC heater 153. Alternatively, as shown in Fig.20, the PTC heater 153 may be so shaped that it substantially encloses the microchip 100 but is partially open.

That is, the shape of the PTC heater 153 is not necessarily intrinsically planar but may also be a parallelepiped or the like three-dimensional structure that encloses the microchip 100 in its inside. That is, it is sufficient that the PTC heater 153 has the function of supplying heat to the capillaries 116 and maintaining the capillaries at an even temperature.

It is also possible to provide a configuration in which a heat conductive plate is disposed between the microchip 100 and the PTC heater 153 so that these will be in contact with the conductive plate, though such configuration is not shown in Fig.19 or Fig.20.

The present Application asserts priority rights based on the previous patent application filed in Japan. The total contents of the disclosure of the previous patent application are to be construed to be incorporated by reference into the present Application. The matter disclosed in the previous patent application, including the claims, specification and drawings, as the basis of assertion of the priority rights, is to be construed to have been stated at the filing data of the previous patent application, as the date of assertion of the priority rights, without being affected by the matter stated herein, that is, as if the present description has not been in existence.

Part or all of the above described exemplary embodiments may non-restrictively be expressed as following modes, but not limited thereto.

### [Mode 1]

Same as the electrophoresis apparatus according to the above mentioned first aspect.

### [Mode 2]

The electrophoresis apparatus according to mode 1, wherein.
the membrane heater includes a current limiting element whose resistance value increases as the current flows therethrough; and an electrode interconnect extending in the first direction and configured for supplying power to the current limiting element.

### [Mode 3]

The electrophoresis apparatus according to mode 1, wherein,
the membrane heater includes a current limiting element whose resistance value increases as the current flows therethrough; and an electrode interconnect extending in a second direction perpendicular to the first direction and configured for supplying power to the current limiting element.

### [Mode 4]

The electrophoresis apparatus according to any one of modes 1 to 3, further comprising
a heat conductive plate arranged intermediate between the microchip and the membrane heater.

### [Mode 5]

The electrophoresis apparatus according to any one of modes 1 to 4, wherein,
the membrane heater includes a PTC (positive temperature coefficient) element having a self-temperature control function.

### [Mode 6]

Same as the method for electrophoresis according to the above mentioned second aspect.

### [Mode 7]

The electrophoresis apparatus according to any one of modes 1 to 5, wherein,
the microchip includes a PCR section in which a desired region in DNA is amplified ;
a denaturing section in which amplicon amplified in the PCR section is denatured from double-strand DNA into single-strand DNA; and
an electrophoresis section in which the amplicon is separated based on the length of sequence.

### [Mode 8]

The electrophoresis apparatus according to mode 7, wherein
the amplicon amplified by the PCR section is allowed to flow into the denaturing section along with a keeping reagent.

### [Mode 9]

The electrophoresis apparatus according to mode 7 or 8, wherein,
the denaturing section has a temperature adjustment section that adjusts the temperature in the denaturing section to a temperature of denaturing amplicon as a double strand DNA into a single strand DNA.

### [Mode 10]

The electrophoresis apparatus according to any one of modes 7 to 9, further comprising
a volume determination section having a capacity smaller than that of the PCR section.

### [Mode 11]

The electrophoresis apparatus according to any one of modes 7 to 10, wherein,
the PCR reagent comprising the amplicon and the keeping agent are mixed at a mixing ratio of 1:2 to 1:9 in the denaturing section.

### [Mode 12]

The electrophoresis apparatus according to any one of modes 7 to 11, wherein,
the keeping agent is formamide.

The disclosures of the above mentioned non-Patent Literatures and so forth are to be incorporated herein by reference. The exemplary embodiments or Examples may be modified or adjusted within the concept of the entire disclosures of the present invention, inclusive of claims, based on the fundamental technical concept of the invention. A series of combinations or selections of elements herein disclosed (elements of claims, Examples and drawings) may be made within the context of the claims of the present invention. That is, the present invention may include a wide variety of changes or corrections that may occur to those skilled in the art in accordance with the total disclosures inclusive of the claims and the drawings as well as the technical concept of the invention. In particular, it should be understood that any optional numerical figures or sub-ranges contained in the ranges of numerical values set out herein ought to be construed to be specifically stated even in the absence of explicit statements.

### REFERENCE SIGNS LIST

- 10: DNA analysis apparatus
- 11: base member
- 12: table
- 13, 14: temperature adjustment units
- 15: electrophoresis unit
- 16: lid
- 17: hinge
- 18a, 18b: pins
- 19a, 19b: pin holes
- 20: set of electrodes
- 21: pressurizing hole
- 22: tubes
- 23: solenoid valve
- 24: pressure accumulator
- 25: controller
- 26: electromagnet
- 27: power supply unit
- 28: DNA analysis unit
- 100, 201: microchips
- 101: sample solution injection section
- 102: wash buffer injection section
- 103: PCR reagent injection section
- 104: formamide injection section
- 105: electrophoresis polymer injection section
- 106: drainage port
- 111: DNA extraction section
- 112: PCR section
- 113: volume determination section
- 114: denaturing section
- 115: electrophoresis section
- 116, 202: capillaries
- 117: electrode chamber
- 131, 131a: heat conductive material
- 132, 132a: temperature sensors
- 133: Peltier element
- 134: heat dissipation plate
- 151, 203: heat conductive plate
- 152: measurement hole
- 153: PTC heater
- 154: laser output unit
- 155: light receiving unit
- 156: resin member
- 157: PTC element
- 158a anode: interconnect
- 158b: cathode interconnect
- 159: region of PTC heater
- 160: region of conductive plate
- 200: flow path
- 204: nichrome wire
- 301: membrane heater
- 302: controller

## Claims

1. An electrophoresis apparatus, comprising:
a membrane heater supplied with power from a power supply and having a self-temperature control function; and
a controller that, at the time of electrophoresis with at least one capillary extending inside of a microchip in a first direction, controls the supply of power from the power supply to the membrane heater;
wherein, the membrane heater is arranged so as to supply heat to the capillary to keep the capillary at an even temperature.

2. The electrophoresis apparatus according to claim 1, wherein,
the membrane heater comprises a current limiting element whose resistance value increases as the current flows therethrough; and
an electrode interconnect extending in the first direction and configured to supply power to the current limiting element.

3. The electrophoresis apparatus according to claim 1, wherein,
the membrane heater comprises a current limiting element whose resistance value increases as the current flows therethrough; and
an electrode interconnect extending in a second direction perpendicular to the first direction and configured to supply power to the current limiting element.

4. The electrophoresis apparatus according to any one of claims 1 to 3, further comprising
a heat conductive plate interposed between the microchip and the membrane heater.

5. The electrophoresis apparatus according to any one of claims 1 to 4, wherein,
the membrane heater is configured by comprising a PTC (positive temperature coefficient) element having a self-temperature control function.

6. A method for electrophoresis using a microchip comprising electrode chambers into which electrodes are introduced and at least one capillary communicated with the electrode chambers, the method comprising the steps of:
applying a dc current via the electrode chambers to a target of analysis; and
supplying a power to a membrane heater which has a self-temperature controlling function and is arranged so as to supply heat to the capillary to keep the capillary at an even temperature.

7. An electrophoresis apparatus, comprising:
a heater supplied from a power supply with power and having a self-temperature controlling function; and
a control unit that, at the time of electrophoresis with at least one capillary extending inside of a microchip in a first direction, controls supply of power from the power supply to the heater;
wherein the heater being arranged so as to supply heat to the capillary to keep the capillary at an even temperature.

8. The electrophoresis apparatus according to claim 7, wherein,
the heater comprises a current limiting element whose resistance value increases as the current flows therethrough; and
an electrode interconnect extending in the first direction and configured to supply power to the current limiting element.

9. The electrophoresis apparatus according to claim 7, wherein,
the heater comprises a current limiting element whose resistance value increases as the current flows therethrough; and
an electrode interconnect extending in a second direction perpendicular to the first direction and configured to supply power to the current limiting element.

10. The electrophoresis apparatus according to any one of claims 7 to 9, further comprising
a heat conductive plate interposed between the microchip and the heater.

11. The electrophoresis apparatus according to any one of claims 7 to 10, wherein,
the heater is configured by comprising a PTC (positive temperature coefficient) element having a self-temperature control function.

12. A method for electrophoresis using a microchip comprising electrode chambers into which electrodes are introduced and at least one capillary communicated with the electrode chambers, the method comprising the steps of:
applying a dc current via the electrode chambers to a target of analysis; and
supplying power to a heater which is arranged so as to supply heat to the capillary to keep the capillary at an even temperature, and having a self-temperature controlling function.
